(19) Europäisches Patentamt
European Patent Office
Office européen des brevets

(11) Publication number: 0 342 396 A1

# (12) EUROPEAN PATENT APPLICATION

(21) Application number: 89107480.9

(51) Int. Cl.[4]: A61K 9/06 , A61K 31/48

(22) Date of filing: 25.04.89

(30) Priority: 09.05.88 YU 898/88

(43) Date of publication of application: 23.11.89 Bulletin 89/47

(84) Designated Contracting States: AT BE CH DE FR GB IT LI NL

(71) Applicant: LEK, tovarna farmacevtskih in kemicnih izdelkov, n.sol.o
Verovskova 57
61000 Ljubljana(YU)

(72) Inventor: Djordjevic, Nebojsa, Dr.
Rimska 12
YU-61000 Ljubljana(YU)
Inventor: Kofler, Bojan
Podlubnik 301
YU-64220 Skofka Loka(YU)
Inventor: Kramaric, Anton
Scopolijeva 16/E
YU-61000 Ljubljana(YU)
Inventor: Lavric, Bogomila
Pavsiceva 4
YU-61000 Ljubljana(YU)
Inventor: Vene-Mozina, Angela
Bratovzeva ploscad 20
YU-61000 Ljubljana(YU)
Inventor: Vukmirovic, Andreja
Dergomaska 16
YU-61000 Ljubljana(YU)
Inventor: Grosman, Polona
Verovskova 54
YU-61000 Ljubljana(YU)
Inventor: Zorz, Miran, M.Sc.
Presernova 53
YU-61290 Grosuplje(YU)
Inventor: Stimac, Zorka
Narodne zascite 15
YU-61000 Ljubljana(YU)
Inventor: Meden, Breda
Bratovzeva ploscad 25
YU-61000 Ljubljana(YU)
Inventor: Zagar, Olga
Prijateljeva 19
YU-61000 Ljubljana(YU)

(74) Representative: Patentanwälte Deufel- Schön- Hertel- Lewald- Otto
Isartorplatz 6 PO Box 260247
D-8000 München 26(DE)

## (54) Ophthalmic ointments for the treatment of glaucoma, comprising ergot alkaloids.

(57) There are described a novel stable ophthalmic ointment for the treatment of increased intraocular pressure in glaucoma patients, comprising a therapeutically effective amount of a peptidic ergot alkaloid of the formula

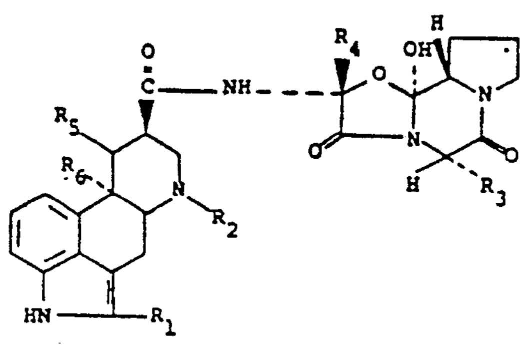

EP 0 342 396 A1

Xerox Copy Centre

wherein
$R_1$ is hydrogen or halogen,
$R_2$ is hydrogen or alkyl having 1 to 4 carbon atoms, and
(i)
$R_3$ is iscpropyl, sec. butyl or isobutyl,
$R_4$ is methyl, ethyl or isopropyl, and
$R_5$ is hydrogen and $R_6$ is hydrogen or methoxy or $R_5$ and $R_6$ together are a single bond, or
(ii)
$R_3$ is benzyl, $R_4$ is methyl, $R_5$ is hydrogen and $R_6$ is hydrogen or methoxy,
in the form of free base or in the form of a pharmaceutically acceptable acid addition salt, in a concentration between 0.005 and 0.05 % by weight in a topically applicable ophthalmic pharmaceutically acceptable carrier and other excipients, the use thereof in a method for the treatment of increased intraocular pressure in glaucoma patients and a process for preparing the same.

# OPHTHALMIC OINTMENTS FOR THE TREATMENT OF GLAUCOMA, COMPRISING ERGOT ALKALOIDS

## Technical Field of the Invention

The present invention belongs to the field of pharmaceutical industry and relates to ophthalmic ointments for the treatment of increased intraocular pressure in glaucoma patients, which ointments comprise peptidic ergot alkaloids.

According to the present invention, from the group of peptidic ergot alkaloids there are preferably used dihydroegotoxine (i.e. a mixture of dihydroergocristine, dihydroergocornine and a 2:1 mixture of alpha- and beta-dihydroergocryptine), bromocryptine and dihydroergotamine, either in the form of free bases or in the form of acid addition salts such as methanesulfonates (mesylates), maleates or tartrates.

By formulating these ergot alkaloids into ophthalmic ointment forms there is achieved a high stability of topical ophthalmic medication in the treatment of glaucoma. Upon the application of such a medication, the intraocular pressure (I.O.P.) is considerably reduced.

## Technical Problem

There exists a constant need for novel, stable, non-irritant galenic forms of known active substances for the treatment of increased intraocular pressure in glaucoma patients, whereby there would be achieved good results without side effects by simple application once or twice a day. Of particular interest are such forms with peptidic ergot alkaloids such as dihydroergotoxine, bromocryptine or dihydroergotamine (all in the form of the methanesulfonate salts), which are known to have low toxicity.

## Prior Art

Glaucoma is an ophthalmic disorder characterized by increased intraocular pressure. There exists an imbalance between production and outflow of the aqueous humor. Untreated disease ultimately results in irreversible damage to the retina and in blindness. It has been known to treat glaucoma with the miotic pilocarpine, which must be applied topically several times a day. This treatment, however, might result in painful eye irritation and may have other side effects. In the case of a prolonged treatment with pilocarpine, increased doses of pilocarpine, i.e. concentrations up to 4 %, become necessary to maintain the efficiency of the drug. In many cases pilocarpine wholly loses its efficacy after prolonged treatment.

The effect of some hydrogenated ergot alkaloids upon glaucoma is known and described in the literature (A. Posner, Amer. J. Ophthal. 33, 1551-54 (1950) and other).

Dihydroergotoxine methanesulfonate is a sympatholytic active substance, consisting of equal parts of dihydroergocristine, dihydroergocornine and of a 2:1 mixture of alpha- and beta-dihydroergocryptine (The Merck Index, 10th Edition (1983) 3596). In the treatment of glaucoma this drug (Hydergine[R]) was administered parenterally, intravenously and subcutaneously; the results, however, were poor and inconsistent, the decrease of the eyeball tension was related to the decrease of the general blood pressure. M. Diotallevi and G. Auricchio, Ophthalmologica 147, 448-454 (1964), studied the effect of topically applied 0.1 % aqueous solution of dihydroergotoxine methanesulfonate (Hydergine[R]) upon eyeball tension. They found that the instillation of the active substance in the conjunctival sac causes a significant decrease of the intraocular pressure (I.O.P.) in rabbit and human eyes. Dihydroergotoxine methanesulfonate acts as an agonist as well as an antagonist on the neurotransmitter receptors, which regulate the production and the outflow of the aqueous humor, thus reducing the intraocular pressure.

Dihydroergotoxine methanesulfonate in the form of eyedrops, however, ist not suitable for the treatment of glaucoma since in the solution it is stable only in low pH ranges (3.2), which are physiologically unsuitable. Eyedrops and hydrogels to be applied on the eye must have a pH value within the physiologically acceptable range. The reaction of dihydroergotoxine methanesulfonate in the employed concentration is too acid, which would irritate the eye; at higher pH values, however, the preparation is unstable. Therapeutical efficacy of eyedrops can only be achieved with active substances having good bioavailability or by very frequent instillation thereof. Since the permeability of the corneal epithelium for peptidic ergot alkaloids is low, the suitable medication dosage form in this case is an ophthalmic ointment, which owing to its physiological, chemical and rheological properties greatly increases the time of stay of the active compound in the conjunctival sac.

Bromocryptine (generic name for 2-bromo-alpha-ergocryptine, commercially available in the form of its methanesulfonate salt, Merck Index, 10th Edition, 1386) is a well-known dopaminergic drug. Recently, there were reported the possibility of using bromocryptine for reducing intraocular pressure in rabbits by its topical instillation into the eye in a concentration of from 0.01 to 1.0 % (Curr. Eye Res., 2(5). 281-288 (1981-82)) as well as a considerable decrease of intraocular pressure without any change of pupillary diameter when used on volunteers in a dose of 1.25 mg (Mekki et al., Lancet 1983/I, 1250-1251). In spite of the potential applicability of bromocryptine in the treatment of glaucoma, its practical application has been seriously hindered by the lack of a suitable dosage form for this indication. When administered orally, bromocryptine acts to a considerable extent as a dopaminergic agent as well as an inhibitor of the prolactine secretion activity, which is undesirable in patients only requiring the treatment of glaucoma.

It is known that bromocryptine and its acid addition salts, such as the methanesulfonate (mesylate) salt, are poorly soluble in media that are suitable for the instillation into the eye in the form of eyedrops. Owing to the instability of such forms, bromocryptine in the form of the free base or of acid addition salts separates from the solution; hence such solutions for the treatment of glaucoma must be prepared immediately before their use.

An important solution of this problem is described in USP 4,654,345, wherein there is disclosed the preparation of lyophilisates comprising bromocryptine or an acid addition salt thereof, benzalkonium chloride as a stabilizer and a polymeric matrix material for the lyophilisate. The lyophilisate comprising bromocryptine or an acid addition salt thereof as the active ingredient is capable of reconstitution into an isotonic medium to provide a stable formulation for ocular instillation, suitable for the treatment of glaucoma. Such liquid forms are stable up to about 3 weeks at 5 °C and up to 10 days at ambient temperature without any visible decomposition or precipitation. For the reconstitution of the lyophilisates into the isotonic medium there is described the possible use of dispenser devices, which make possible the preparation of calculated unit doses of the lyophilisate and isotonic medium for later reconstitution. The drawbacks of this method are the use of expensive lyophilisation technique, the necessary manufacture of special dispenser devices containing calculated unit doses of the lyophilisate and the isotonic medium and, finally, the administration of the prepared ophthalmic solution, which is only stable up to 3 weeks, several times a day.

## Description of the Solution of the Technical Problem with Examples of Embodiment

The present invention is based on the problem how to prepare a stable, non-irritant galenic form for the treatment of increased intraocular pressure in glaucoma patients, comprising peptidic ergot alkaloids of the formula

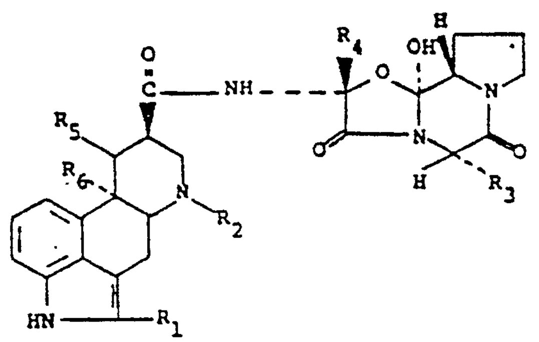

wherein

$R_1$ is hydrogen or halogen,

$R_2$ is hydrogen or alkyl having 1 to 4 carbon atoms, and

(i)

$R_3$ is isopropyl, sec. butyl or isobutyl,

$R_4$ is methyl, ethyl or isopropyl, and

$R_5$ is hydrogen and $R_6$ is hydrogen or methoxy or $R_5$ and $R_6$ together are a single bond, or

(ii)

$R_3$ is benzyl, $R_4$ is methyl, $R_5$ is hydrogen and $R_6$ is hydrogen or methoxy,

in the form of free base or in the form of a pharmaceutically acceptable acid addition salt, particularly

dihydroergotoxine, bromocryptine or dihydroergotamine, which could be prepared in a fast, inexpensive and simple way and and easily administered in the eye once or twice a day.

This aim is achieved by ophthalmic ointments comprising a therapeutically active amount of a peptidic ergot alkaloid in a pharmaceutically acceptable base (carrier) and other excipients.

Ophthalmic ointments are a galenic form for a topical administration of the active substance on/in the eye. They are applied in the conjunctional sac or on the edge of the eyelid. In Dolder-Skinner, Ophthalmika, Pharmakologie, Biopharmazie und Galenik der Augenarzneimittel, 3. Auflage, Wissenschaftliche Verlagsgesellschaft, Stuttgart 1983, or John. W. Shell, The Pharmacokinetics of Topical Glaucoma Medications (9), Edited by Stephen M. Drance, M.D., 1984 by Grune & Strattor, Inc., there are reported the advantages of ophthalmic ointments over eyedrops: owing to the sustained release of the active drug, the former make possible a protracted activity of the drug and thereby reduce the frequency of its administration. Likewise, the bioavailability, expressed as the area un der the time-active substance release curve, is significantly greater than the bioavailability of the active substance in aqueous solutions or suspensions.

Opthalmic ointments, which are the object of the present invention, are prepared in a known way. First, from known bases (carriers) for ophthalmic ointments, such as liquid paraffin, white vaseline, lanolin, there is prepared a homogeneous, sterile ophthalmic ointment base, which under the addition of a stabilizer, such as butyl-hydroxyanisole in liquid paraffin, is subjected to dry sterilization at a temperature of about 140 °C, then cooled to about 40 °C and subsequently blended with a homogeneous sterile suspension of butylhydroxyanisole and micronized peptidic ergot alkaloid of the above formula, such as dihydroergotoxine, bromocryptine or dihydroergotamine (all in the form of their methanesulfonate salts). The thus prepared ointment is cooled to room temperature and filled into tubes, sterilized with ethylene oxyde, under sterile conditions.

The maximum admissible water content of the base is 5 %. As active substances there are used micronized dihydroergotoxine methanesulfonate, bromocryptine methanesulfonate and dihydroergotamine methanesulfonate with a particle size of less than 40 µm with 90 % of the particles having a size of less than 20 µm.

The opthalmic ointment of the invention comprises from 0.005 to 0.05 % by weight of the peptidic ergot alkaloid of the above formula, such as dihydroergotoxine, bromocryptine or dihydroergotamine or methanesulfonate salts thereof. In addition, the ophthalmic ointment comprises from 60 to about 99.99 % by weight of hydrocarbons, such as liquid paraffin, white vaseline, as topically applicable ophthalmic pharmaceutically acceptable carrier, from 0 to 40 % by weight of lanolin and from 0.001 to 0.05 % by weight of a stabilizer, such as butyl-hydroxyanisole.

The thus prepared galenic form meets the requirements for ophthalmic ointments, such as sterility, non-irritability, efficacy, capacity of adequate distribution of active substances and solutions thereof, spreadability, capacity of quick distribution across the eyeball in the form of a thin film, good adherence, stability and small interference with vision.

An ocular irritation test for ophthalmic ointments was performed on albino rabbits with 0.01 %, 0.025 %, 0.05 % and 0.4 % dihydrotoxine methanesulfonate or bromocryptine methanesulfonate and with a placebo. The results showed that no major irritative changes were to be detected with either active substance at concentrations of 0.005 and 0.05 % by weight nor with the placebo; at concentrations, however, of 0.1 % by weight and more there occurs irritation.

The clinical tests on glaucoma patients were performed with an ophthalmic ointment comprising 0.01 % and 0.025 % of dihydroergotoxine methanesulfonate or bromocryptine methanesulfonate. The results are shown in Tables 1-4. The best results in glaucoma patients were obtained with an ointment comprising 0.025 % of the active substance. At this concentration, dihydroergotoxine methanesulfonate and bromocryptine methanesulfonate cause a reduction of intraocular pressure of 38 % and 30 %, respectively. Comparative tests were made with dihydroergotoxine (base), which showed lower results, and with a placebo. In no patient undesired effects were observed. The stability of the ophthalmic ointment comprising dihydroergotoxine methanesulfonate or bromocryptine methanesulfonate was tested in accordance with the method for testing creams, emulsions, gels and ophthalmic ointments as described in the pub lication by W. Grimm and Schepky, Stabilitaetspruefung in der Pharmazie, Editio Cantor, Aulendorf, 297-301 (1980). On the basis of the results of accelerated six-month stability tests of the ophthalmic ointment comprising 0.025 % of dihydroergotoxine methanesulfonate or bromocryptine methanesulfonate, a three-year stability of the ointment at a temperature up to 25 °C can be assumed.

The invention is illustrated, but in no way limited by the following Examples of embodiment.

**Example 1**

Ophthalmic ointment comprising 0.025 % of dihydroergotoxine

| Ingredients | % by weight |
|---|---|
| dihydroergotoxine methanesulfonate | 0.025 |
| butyl-hydroxyanisole | 0.005 |
| lanolin | 10.000 |
| Vaselinum album (white vaseline) | 80.000 |
| Paraffinum liquidum (liquid paraffin) | 9.970 |

Method of preparation

90 g of liquid paraffin, 800 g of white vaseline, 100 g of lanolin and 0.050 g of butyl-hydroxyanisole, dissolved in 9.7 g of liquid paraffin, were sterilized at the temperature of 140 °C for 3 hours in a hot-air sterilizer.

The sterilized ingredients of the ointment base (white vaseline, lanolin and liquid paraffin) were cooled to 40 °C and worked up under sterile conditions into a homogeneous ointment base.

The sterilized solution of butyl-hydroxyanisole and 0.250 g of sterile micronized dihydroergotoxine methanesulfonate having a particle size of less than 40 µm with 90 % of the particles having a size of less than 20 µm were worked up under sterile conditions into a suspension, which was homogeneously blended with the ointment base (carrier). The thus prepared ophthalmic ointment was cooled to room temperature and put into tubes, sterilized with ethylene oxide, under sterile conditions.

**Example 2**

Ophthalmic ointment comprising 0.025 of dihydroergotoxine

| Ingredients | % by weight |
|---|---|
| dihydroergotoxine methanesulfonate | 0.025 |
| butyl-hydroxyanisole | 0.005 |
| Vaselinum album | 65.000 |
| Paraffinum liquidum | 34.970 |

The method of preparation was the same as in Example 1.

**Example 3**

Ophthalmic ointment comprising 0.025 % of dihydroegotoxine

| Ingredients | % by weight |
|---|---|
| dihydroergotoxine (base) | 0.025 |
| butyl-hydroxyanisole | 0.005 |
| lanolin | 10.000 |
| Vaselinum album | 80.000 |
| Paraffinum liquidum | 9.970 |

The method of preparation was the same as in Example 1.

**Example 4**

Ophthalmic ointment comprising 0.025 % od dihydroergotoxine

| Ingredients | % by weight |
|---|---|
| dihydroergotoxine (base) | 0.025 |
| butyl-hydroxyanisole | 0.005 |
| Paraffinum liquidum | 34.970 |
| Vaselinum album | 65.000 |

The method of preparation was the same as in Example 1.

**Example 5**

Ophthalmic ointment comprising 0.025 % of bromocryptine

| Ingredients | % by weight |
|---|---|
| bromocryptine methanesulfonate | 0.025 |
| butyl-hydroxyanisole | 0.005 |
| Paraffinum liquidum | 34.970 |
| Vaselinum album | 65.000 |

The method of preparation was the same as in Example 1, except that micronized sterile bromocryptine methanesulfonate was used as the active substance.

**Example 6**

Ophthalmic ointment comprising 0.01 % of bromocryptine

| Ingredients | % by weight |
|---|---|
| bromocryptine methanesulfonate | 0.010 |
| butyl-hydroxyanisole | 0.005 |
| Paraffinum liquidum | 34.985 |
| Vaselinum album | 65.000 |

The method of preparation was the same as in Example 1, except that micronized sterile bromocryptine methanesulfonate was used as the active substance.

**Example 7**

Ophthalmic ointment comprising 0.010 of dihydroergotoxine

| Ingredients | % by weight |
|---|---|
| dihydroergotoxine methanesulfonate | 0.010 |
| butyl-hydroxyanisole | 0.005 |
| Vaselinum album | 65.000 |
| Paraffinum liquidum | 34.985 |

The method of preparation was the same as in Example 1.

**Example 8**

Ophthalmic ointment comprising 0.025 % of dihydroergotamine

| Ingredients | % by weight |
|---|---|
| dihydroergotamine methanesulfonate | 0.025 |
| butyl-hydroxyanisole | 0.005 |
| Vaselinum album | 65.000 |
| Paraffinum liquidum | 34.970 |

The method of preparation was the same as in Example 1, except that micronized sterile dihydroegotamine methanesulfonate was used as the active substance.

**Example 9**

Ocular Irritation Test

There were tested ophthalmic ointments comprising
0.01 %, 0.025 % and 0.4 % of dihydroergotoxine methanesulfonate,
0.01 %, 0.025 % and 0.4 % of bromocryptine methanesulfonate, and
a placebo ophthalmic ointment.

For each sample of the ophthalmic ointments 3 albino rabbits without visible eye lesion were used. The

samples were applied into the conjunctional sac of the left eye, the right eye serving as control. The reactions were observed 1, 24, 48 and 72 hours and on the fourth and seventh day after the application.

Subsequently all samples were tested on another group of animals, to which the ointment was applied for 5 consecutive days.

Results:

With either ophthalmic ointment comprising the active substance in concentrations of 0.01 % and 0.025 % as well as with the placebo, no major changes owing to irritation were detected.

In the case of the ophthalmic ointment comprising 0.4 % of the active substance, there were observed changes in the form of red eyes and enlarged veinlets one hour after the application. After 48 hours these changes were still present and were added by a slight swelling of the eyelids.

Both samples comprising 0.4 % of the active substance were then tested on a new group of animals, to which the ointments were applied for 5 consecutive days. Although the changes on the eyes were present all the time, they did not intensify.

It can be concluded that the ophthalmic ointments comprising as active substances dihydroergotoxine methanesulfonate and bromocryptine methanesulfonate within the concentration range between 0.01 and 0.025 % have no irritant effect; higher concentrations, however, act irritantly. On the other hand, said active substances can be used in higher concentrations to increase blood circulation.

**Example 10**

Clinical trials

Effect of ophthalmic ointment comprising 0.01 % of dihydroergotoxine methanesulfonate or bromocryptine methanesulfonate on the intraocular pressure (I.O.P.) in glaucoma patients

The effect of the ophthalmic ointment comprising 0.01 % of dihydroergotoxine (as base and as methanesulfonate salt) and of bromocryptine methanesulfonate on the intraocular pressure in both eyes was tested in five male and three female glaucoma patients aged from 34 to 62 years. In contrast to the placebo, the ophthalmic ointment comprising 0.01 % of said active compounds produced a visible reduction of I.O.P. in both eyes with a tendency of further reduction of pressure.

In the case of the ophthalmic ointment comprising dihydroergotoxine methanesulfonate, a reduction of I.O.P. by 18 % took place within a few hours, while in the case of the ointment comprising bromocryptine methanesulfonate, the maximum reduction of I.O.P. by 21 % was reached as early as I hour after the application. This effect was present for at least five days. Dihydroergotoxine base reduced the I.O.P. to a somewhat lesser extent than the corresponding acid addition salt.

A reduction of the I.O.P. was observed in all glaucoma patients. No undesired side effects were observed in any patient.

The results of the trials are shown in Tables 1 and 2.

TABLE 1

| Effects of the Treatment with an Ophthalmic Ointment on Left-Eye I.O.P. Values in Glaucoma Patients | | | | | | |
|---|---|---|---|---|---|---|
| Kind of Ophthalmic Ointment | Value before Medication | INTRAOCULAR PRESSURE (kPa) | | | | |
| | | Values during the Medication | | | | |
| | | 1st day | 2nd day | 3rd day | 4th day | 5th day |
| Placebo | 3.30±0.11 | 3.33±0.05 (101.0 %) | 3.50±0.12 (106.7 %) | 3.43±0.15 (103.7 %) | 3.45±0.03 (104.9 %) | |
| 0.01 % dihydroergotoxine (base) | 3.48±0.18 | 3.40±0.21 (97.7 %) | 3.25±0.28 (93.2 %) | 3.18±0.28 (90.9 %) | 3.18±0.28 (90.9 %) | 3.18±0.28 (90.9 %) |
| 0.01 % dihydroergotoxine methanesulfonate | 3.57±0.17 | 3.34±0.35 (93.1 %) | 3.30±0.23 (92.4 %) | 3.24±0.35 (90.2 %) | 2.80±0.21 (78.7 %) | 2.80±0.21 (78.7 %) |
| 0.01 % bromocryptine methanesulfonate | 3.37±0.28 | 2.64±0.24 (79.0 %) | 2.74±0.22 (82.5 %) | 2.77±0.22 (83.0 %) | 2.70±0.22 (81.0 %) | 2.73±0.25 (81.5 %) |
| The results represent the mean values ± the mean value errors. | | | | | | |

TABLE 2

| Effects of the Treatment with an Ophthalmic Ointment on Right-Eye I.O.P. Values in Glaucoma Patients | | | | | | |
|---|---|---|---|---|---|---|
| Kind of Ophthalmic Ointment | Value before Medication | INTRAOCULAR PRESSURE (kPa) | | | | |
| | | Values during the Medication | | | | |
| | | 1st day | 2nd day | 3rd day | 4th day | 5th day |
| Placebo | 3.43±0.10 (100 %) | 3.48±0.09 (101.5 %) | 3.50±0.08 (102.4 %) | 3.60±0.06 (105.3 %) | 3.43±0.10 (100.0 %) | |
| 0.01 % dihydroergotoxine (base) | 4.00±0.35 (100 %) | 4.00±0.35 (100.0 %) | 3.63±0.24 (91.2 %) | 3.88±0.31 (97.2 %) | 3.83±0.27 (96.3 %) | 3.83±0.27 (96.3 %) |
| 0.01 % dihydroergotoxine methanesulfonate | 4.08±0.71 (100 %) | 3.90±0.62 (96.2 %) | 3.78±0.67 (92.4 %) | 3.78±0.56 (93.5 %) | 3.15±0.16 (82.1 %) | 3.65±0.57 (90.3 %) |
| 0.01 % bromocryptine methanesulfonate | 3.88±0.32 (100 %) | 3.25±0.34 (83.2 %) | 3.55±0.46 (90.0 %) | 3.30±0.39 (84.1 %) | 3.17±0.30 (81.3 %) | 3.27±0.33 (83.8 %) |
| The results represent the mean values ± the mean value errors. | | | | | | |

**Example 11**

Clinical trials

Effect of ophthalmic ointment comprising 0.025 % of dihydroergotoxine methanesulfonate or bromocryptine methanesulfonate on the intraocular pressure (I.O.P) in glaucoma patients

The effect of the ophthalmic ointment comprising 0.025 % of dihydroergotoxine methanesulfonate or bromocryptine methanesulfonate on the intraocular pressure in both eyes was tested in four male and three female glaucoma patients, agend from 31 to 64 years.

The ointment was applied into the conjunctival sac in the morning and in the evening. Before application, the I.O.P. was measured. This procedure was repeated for five days.

In the group where the placebo ophthalmic ointment was applied, no changes in I.O.P. were observed. In patients, who received the ointment comprising dihydroergotoxine methanesulfonate into the conjunctival sac, the I.O.P. gradually decreased from 4.00 kPa to 2.53 kPa, while in the case of the ointment comprising bromocryptine methanesulfonate, the I.O.P. decreased from an average 4.00 kPa to 2.67 kPa.

From the results of testing as shown in Tables 3 and 4 it can be seen that the ophthalmic ointment comprising 0.025 % of dihydroergotoxine methanesulfonate reduced the I.O.P. in glaucoma patients by 38 % and the ointment comprising bromocryptine methanesulfonate by 30 %. No undesired side effects were observed in any patient.

TABLE 3

Effects of the Treatment with an Ophthalmic Ointment on Right-Eye I.O.P. Values in Glaucoma Patients

| Kind of Ophthalmic Ointment | Value before Medication | INTRAOCULAR PRESSURE (kPa) Values during the Medication | | | | |
|---|---|---|---|---|---|---|
| | | 1st day | 2nd day | 3rd day | 4th day | 5th day |
| Placebo | 3.30±0.13 | 3.17±0.16 (96.20 %) | 3.27±0.12 (99.27 %) | 3.25±0.09 (98.63 %) | 3.25±0.09 (98.46 %) | 3.30±0.10 (100.09 %) |
| 0.025 % dihydroergotoxine methanesulfonate | 4.24±0.36 | 3.50±0.34 (82.46 %) | 2.84±0.18 (67.00 %) | 2.74±0.11 (64.55 %) | 2.66±0.19 (62.73 %) | 2.63±0.15 (61.97 %) |
| 0.025 % bromocryptine methanesulfonate | 3.97±0.33 | 3.28±0.23 (82.55 %) | 3.06±0.37 (77.11 %) | 2.97±0.32 (74.70 %) | 2.91±0.30 (73.36 %) | 2.82±0.30 (71.07 %) |

The results represent the mean values ± the mean value errors.

TABLE 4

Effects of the Treatment with an Ophthalmic Ointment on Left-Eye I.O.P. Values in Glaucoma Patients

| Kind of Ophthalmic Ointment | Value before Medication | INTRAOCULAR PRESSURE (kPa) Values during the Medication | | | | |
|---|---|---|---|---|---|---|
| | | 1st day | 2nd day | 3rd day | 4th day | 5th day |
| Placebo | 3.17±0.13 | 3.17±0.13 (100.17 %) | 3.19±0.14 (100.84 %) | 3.12±0.07 (98.45 %) | 3.14±0.12 (99.16 %) | 3.19±0.12 (100.84 %) |
| 0.025 % dihydroergotoxine (base) | 4.12±0.40 | 3.31±0.16 (80.34 %) | 2.84±0.18 (68.95 %) | 2.84±0.18 (68.95 %) | 2.75±0.19 (66.75 %) | 2.64±0.14 (64.17 %) |
| 0.025 % bromocryptine methanesulfonate | 4.32±0.37 | 3.50±0.36 (80.89 %) | 3.33±0.46 (76.94 %) | 2.97±0.32 (68.62 %) | 2.82±0.30 (65.30 %) | 2.78±0.26 (64.30 %) |

The results represent the mean values ± the mean value errors.

**Example 12**

Stability test

Ophthalmic ointment comprising 0.025 % of dihydroergotoxine methanesulfonate

The stability of the ophthalmic ointment was examined in accordance with the method for testing creams, emulsions, gels and ophthalmic ointments, proposed by W. Grimm and Schepky, Stabilitaetspruefung in der Pharmazie, Editio Cantor, Aulendorf, 297-301 (1980).

TABLE 5

| Analysed | Time, months | Storage conditions | | | |
|---|---|---|---|---|---|
| | | 2-8 °C | 20±5 °C | 30±1 °C | 37±1 °C |
| Appearance | 0 | | homogeneous, white ointment | | |
| | 1 | homogeneous, white ointment | unaltered | unaltered | unaltered |
| | 3 | | | | |
| | 6 | | | | |
| Dihydroergotoxine methanesulfonate content (total) | 0 | | 100.0 % | | |
| | 1 | | | 100.0 % | 100.0 % |
| | 3 | 100.0 % | 99.5 % | 97.9 % | 100.0 % |
| | 6 | 98.7 % | 102.3 % | 98.5 % | 97.4 % |
| Allied substances (total) | 0 | - | <0.5 % | - | - |
| | 1 | | - | 0.5-1 % | 1-1.5 % |
| | 3 | | 0.5-1 % | 0.5-1.5% | 2.5-3 % |
| | 6 | | 1 % | 2-2.5 % | 3.5-4 % |
| Ergot alkaloid ratio in dihydroergotoxine methanesulfonate: | | | | | |
| dihydroergocornine methanesulfonate | | | | 34.6 % | |
| dihydroergocryptine methanesulfonate* | | | | 32.1 % | |
| dihydroergocristine methanesulfonate | | | | 33.3 % | |

* The ratio of alpha- and beta-dihydroergocryptine is 2.2:1.

On the basis of the results of a 6-month accelerated stability test of the ophthalmic ointment comprising 0.025 % of dihydroergotoxine methanesulfonate, a 3-year stability at a temperature up to 25 °C can be assumed.

**Example 13**

Stability test

Ophthalmic ointment comprising 0.025 % of bromocryptine methanesulfonate

The stability of the ophthalmic ointment was examined in accordance with the method for testing creams, emulsions, gels and ophthalmic ointments, proposed by W. Grimm and Schepky, Stabilitaetspruefung in der Pharmazie, Editio Cantor, Aulendorf, 297-301 (1980).

TABLE 6

| Analysed | **Time,** months | **Storage conditions** | | | |
|---|---|---|---|---|---|
| | | 2-8 °C | 20±5 °C | 30±1 °C | 37±1 °C |
| Appearance | 0 | | homogeneous, white ointment | | |
| | 1 | homogeneous, white ointment | | | |
| | 3 | | unaltered | unaltered | unaltered |
| | 6 | unaltered | unaltered | unaltered | unaltered |
| Bromocryptine methanesulfonate content | 0 | | 100.0 % | | |
| | 1 | - | - | 100.0 % | 100.0 % |
| | 3 | 100.0 % | 99.9 % | 100.5 % | 101.0 % |
| | 6 | 99.8 % | 100.1 % | 100.0 % | 98.5 % |

On the basis of the results of a 6-month accelerated stability test of the ophthalmic ointment comprising 0.025 % of bromocryptine methanesulfonate, a 3-year stability at a temperature up to 25 °C can be assumed.

## Claims

1. A stable ophthalmic ointment for the treatment of increased intraocular pressure in glaucoma patients, comprising a therapeutically effective amount of a peptidic ergot alkaloid of the formula

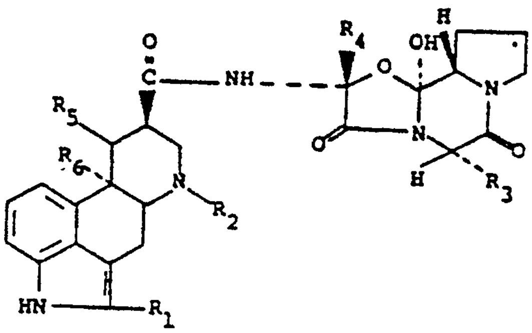

wherein
$R_1$ is hydrogen or halogen,
$R_2$ is hydrogen or alkyl having 1 to 4 carbon atoms, and
(i)
$R_3$ is isopropyl, sec. butyl or isobutyl,
$R_4$ is methyl, ethyl or isopropyl, and
$R_5$ is hydrogen and $R_6$ is hydrogen or methoxy or $R_5$ and $R_6$ together are a single bond, or
(ii)
$R_3$ is benzyl, $R_4$ is methyl, $R_5$ is hydrogen and $R_6$ is hydrogen or methoxy,
in the form of free base or in the form of a pharmaceutically acceptable acid addition salt, in a concentration between 0.005 and 0.05 % by weight in a topically applicable ophthalmic pharmaceutically acceptable carrier and other excipients.

2. A stable opthalmic ointment according to claim 1, comprising as the active compounds dihydroergotoxine methanesulfonate, bromocryptine methanesulfonate or dihydroergotamine methanesulfonate in an ophthalmic pharmaceutically acceptable carrier and other excipients.

3. A stable ophthalmic ointment according to claim 1, comprising as the topically applicable ophthalmic pharmaceutically acceptable carrier from 60 to about 99.99 % by weight of hydrocarbons, such as liquid paraffin, white vaseline, and from 0 to 40 % by weight of lanolin.

4. A stable ophthalmic ointment according to claim 1, comprising from 0.001 to 0.05 % by weight of butyl-hydroxyanisole as a stabilizer for the pharmaceutical preparation.

5. A method for the treatment of increased intraocular pressure in glaucoma patients, comprising the application of an ophthalmic ointment comprising a therapeutically effective amount of a peptidic ergot alkaloid of the formula

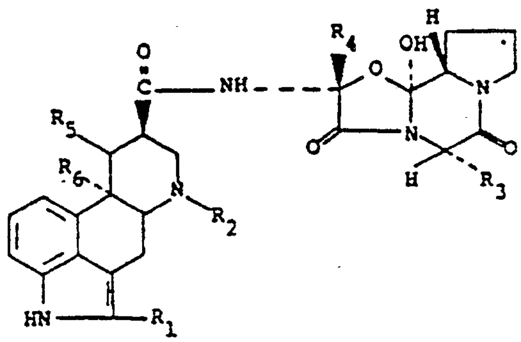

wherein
$R_1$ is hydrogen or halogen,
$R_2$ is hydrogen or alkyl having 1 to 4 carbon atoms, and
(i)
$R_3$ is isopropyl, sec. butyl or isobutyl,
$R_4$ is methyl, ethyl or isopropyl, and
$R_5$ is hydrogen and $R_6$ is hydrogen or methoxy or $R_5$ and $R_6$ together are a single bond, or
(ii)
$R_3$ is benzyl, $R_4$ is methyl, $R_5$ is hydrogen and $R_6$ is hydrogen or methoxy,
in the form of free base or in the form of a pharmaceutically acceptable acid addition salt, in a concentration between 0.005 and 0.05 % by weight in a topically applicable ophthalmic pharmaceutically acceptable carrier and other excipients.

6. A method according to claim 5, comprising the application of an ophthalmic ointment comprising a therapeutically effective amount of dihydroergotoxine methanesulfonate, bromocryptine methanesulfonate or dihydroergotamine methanesulfonate, once or twice a day.

7. A process for preparing a stable ophthalmic ointment for the treatment of increased intraocular pressure in glaucoma patients according to claim 1, characterized in that a mixture of 60 to about 99.99 % by weight of liquid paraffin, white vaseline, optionally from 0 to 40 % by weight of lanolin and from 0.001 to 0.05 % by weight of butyl-hydroxyanisole in liquid paraffin is subjected to dry sterilization at a temperature of about 140 °C, the obtained ointment base is cooled to about 40 °C and subsequently blended with a homogeneous sterile suspension of butyl-hydroxyanisole and micronized peptidic ergot alkaloid of the formula given in claim 1, the prepared ointment is cooled to room temperature and put into tubes under sterile conditions.

8. A process according to claim 7, characterized in that micronized dihydroergotoxine methanesulfonate, bromocryptine methanesulfonate or dihydroergotamine methanesulfonate are used as peptidic ergot alkaloids of the formula given in claim 1.

9. The use of dihydroergotoxine methanesulfonate, bromocryptine methanesulfonate or dihydroergotamine methanesulfonate for preparing a stable ophthalmic ointment for the treatment of increased intraocular pressure in glaucoma patients.

European Patent Office

**PARTIAL EUROPEAN SEARCH REPORT**
which under Rule 45 of the European Patent Convention shall be considered, for the purposes of subsequent proceedings, as the European search report

Application number
EP 89 10 7480

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | LU-A- 86 135 (SANDOZ S.A.)<br>* Claims 1,15; page 5, lines 11-20 *<br>-- | 1-6,9 | A 61 K 9/06<br>A 61 K 31/48 |
| Y | CHEMICAL ABSTRACTS, vol. 87, September 5, 1977, page 333, ref.no. 73330p, Columbus, Ohio, US;<br>A.I. TENTSOVA et al.: "Chemiluminescense method of selecting antioxidants for ointment bases" & FARMATSIYA (MOSCOW) 1977, 26(3), 16-21<br>* Whole abstract *<br>-- | 1-6,9 | |
| X | CHEMICAL ABSTRACTS, vol. 77, July 3, 1972, page 80, ref. no. 775w, Columbus, Ohio, US;<br>J. PECORI-GIRALDI et al.: "Effect of topically administered adrenergic-blocking agents on the intra-<br>./. | | TECHNICAL FIELDS SEARCHED (Int. Cl.4)<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-4,7-9
Claims searched incompletely:
Claims not searched: 5,6
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 06-07-1989 | BERTE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

**PARTIAL EUROPEAN SEARCH REPORT**

Application number EP 89 10 7480



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | ocular pressure" & OPHTHALMOL. PROC. INT. CONGR., 21st 1970 (Pub. 1971). 2, 1147-53<br><br>* Whole abstract *<br><br>-- | 1,9 | |
| X | CHEMICAL ABSTRACTS, vol. 100, April 9, 1984, page 59, ref. no. 114972g, Columbus, Ohio, US; Q.A. MEKKI et al.: "Bromocriptine eyedrops lower intraocular pressure without affecting prolactin levels" & LANCET 1984, 1(8371), 287-8<br><br>* Whole abstract *<br><br>-- | 1,9 | TECHNICAL FIELDS SEARCHED (Int Cl.4) |
| Y | CHEMICAL ABSTRACTS, vol. 98, February 7, 1983, page 363, ref. no. 40598f, Columbus, Ohio, US; & JP-A-57 171 911 (R. TOTANI) 22-10-1982<br><br>* Whole abstract *<br><br>------ | 1-6,9 | |